# EUROPEAN PATENT APPLICATION

(11) **EP 2 554 147 A1**
(43) Date of publication of application: **06.02.2013**
(21) Application number: 11765808.8
(22) Date of filing: 31.03.2011
(51) Int. Cl.: A61F 13/15, A61F 13/49, A61F 13/496, A61F 13/551

(54) **DISPOSABLE UNDERWEAR-TYPE WORN ARTICLE**

(30) Priority: 31.03.2010 JP 2010084583
(71) Applicant: Unicharm Corporation, Ehime 799-0111 (JP)
(72) Inventor: SASAYAMA, Kenichi, Kanonji-shi Kagawa 769-1602 (JP); ICHIKAWA, Makoto, Kanonji-shi Kagawa 769-1602 (JP); YAMAMOTO, Hiroki, Kanonji-shi Kagawa 769-1602 (JP); UKEGAWA, Kazuo, Kanonji-shi Kagawa 769-1602 (JP); NINOMIYA, Akihide, Kanonji-shi Kagawa 769-1602 (JP)
(74) Representative: Peter, Julian
(86) International application number: PCT/JP2011/058345
(87) International publication number: WO 2011/125908

(57) **Abstract**

A disposable pants-type wearing article adapted to prevent body waste from leaking out beyond the peripheries of the leg-openings when disposing of the used article. A disposable pants-type wearing article 1 includes a front panel 106, a rear panel 107 and a central panel 108. When the front panel 106 and the rear panel 107 are respectively folded with respective inner surfaces lying inside, opposite side edges 62, 62 of the central panel 108 get close to each other in a midsection of a crotch region 8 to a distance in a range of 0 to 50 mm.

## Description

### {Technical Field}

The present invention relates to disposable pants-type wearing articles.

### {Background}

Conventionally, pants-type wearing articles such as disposable pants-type diapers are known. Pants-type wearing articles provided with a means to roll up the used articles for disposal thereof in this rolled up state are also known.

For example, the disposable pants-type diaper disclosed in JP 2002-11041A (PTL 1) includes a strip of pressure-sensitive adhesive tape extending in a vertical direction of the diaper. The used diaper soiled with body waste may be rolled up in the vertical direction and then fastened by the strip of pressure-sensitive adhesive tape to prevent the rolled up diaper from being unintentionally unrolled.

The disposable diaper disclosed in JP 2003-19153A (PTL 2) is of pants-type and provided with a strip of pressure-sensitive adhesive tape extending in a transverse direction of the diaper. The used diaper may be rolled up in the transverse direction and then fastened by the strip of pressure-sensitive adhesive tape to prevent the rolled up diaper from being unintentionally unrolled.

### {Citation List}

### {Patent Literature}

{PTL 1}: JP 2002-11041 A
{PTL 2}: JP 2003-19153 A

### {Summary}

### {Technical Problem}

After the pants-type diaper soiled with body waste has been rolled up in the vertical direction or in the transverse direction, some amount of body waste might leak out beyond peripheries of leg-openings.

An object of the present invention is to provide a disposable pants-type wearing article adapted to prevent body waste from leaking out beyond peripheries of leg-openings when disposing of the pants-type wearing article soiled with body waste. {Solution to Problem}

Some embodiments of the present invention provide a disposable pants-type wearing article having a front-back direction, a transverse direction and a vertical direction being orthogonal to one another, and including:
a front panel defining a front waist region, a rear panel defining a rear waist region and a central panel defining a crotch region, wherein these panels are cooperating together to define a pants shape, wherein
each of the panels includes an inner surface and an outer surface opposite to the inner surface,
end segments of the central panel opposite in the front-back direction are bonded to the front panel and the rear panel, respectively, and
the central panel includes a bodily fluid absorbent structure.

In such a wearing article, this invention further includes the following features:
in the opposite end segments of the central panel, opposite side edge portions of the central panel are folded with the inner surface lying inside and bonded to itself;
the respective end segments are at least partially bonded to the front panel and the rear panel, respectively; and
when the front panel and the rear panel are respectively folded along a center line bisecting a dimension in the transverse direction of the wearing article with the inner surfaces lying inside, the opposite side edges of the central panel get close to each other in a midsection of the crotch region until a distance between respective side edges of the opposite side edges is reduced to a range of 0 to 50 mm.

According to one embodiment of this invention, when the front panel and the rear panel are respectively folded along the center line with the inner surfaces lying inside, the respective inner surfaces of the opposite side edge portions of the central panel overlap each other in the crotch region.

According to another embodiment of this invention, the central panel is bonded to the outer surface of at least one of the front and rear panels.

According to even another embodiment of this invention, the central panel bonded to the outer surface has an intermediate segment as viewed in the transverse direction but not bonded to a lower segment as viewed in the vertical direction of at least one of the front and rear panels and this intermediate segment cooperates with at least one of the front and rear panels to define a pocket opening downward as viewed in the vertical direction.

According to yet another embodiment of this invention, the central panel is bonded to the respective inner surfaces of the front and rear panels.

According to still another embodiment of this invention, a distance between the opposite side edges extending along the outer surface of the central panel in the midsection of the crotch region is in a range of 275 to 700 mm.

According to a further embodiment of this invention, the bodily fluid absorbent structure includes an absorbent core in the form of a pad and a water-pervious inner sheet covering at least the upper surface thereof and the absorbent core includes at least superabsorbent polymer particles.

### {Advantageous Effects of Invention}

In the disposable pants-type wearing article according to this invention, the front panel and the rear panel may be respectively folded along the center line bisecting the dimension of the article in the width direction to ensure that the distance between the opposite side edges in the midsection of the crotch region as viewed in the front-back direction is reduced to a range of 0 to 50 mm. From this state, the opposite side edges may be may be overlapped each other with the midsection of the crotch region hanging downward from these opposite side edges to ensure that body waste gathers in the central bottom of this hanging down crotch region. In this way, body waste should not leak out beyond any of the opposite side edges.

### {Brief Description of Drawings}

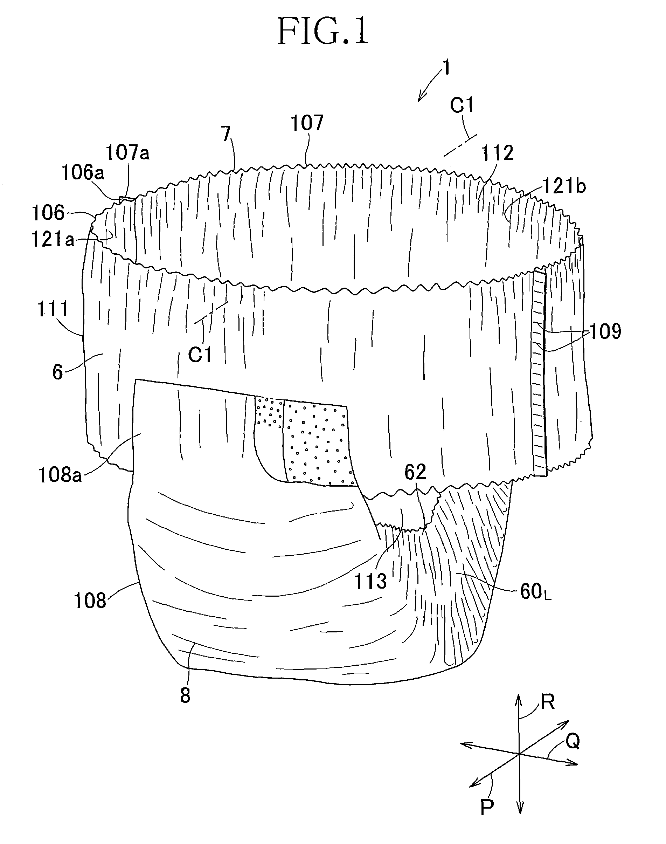

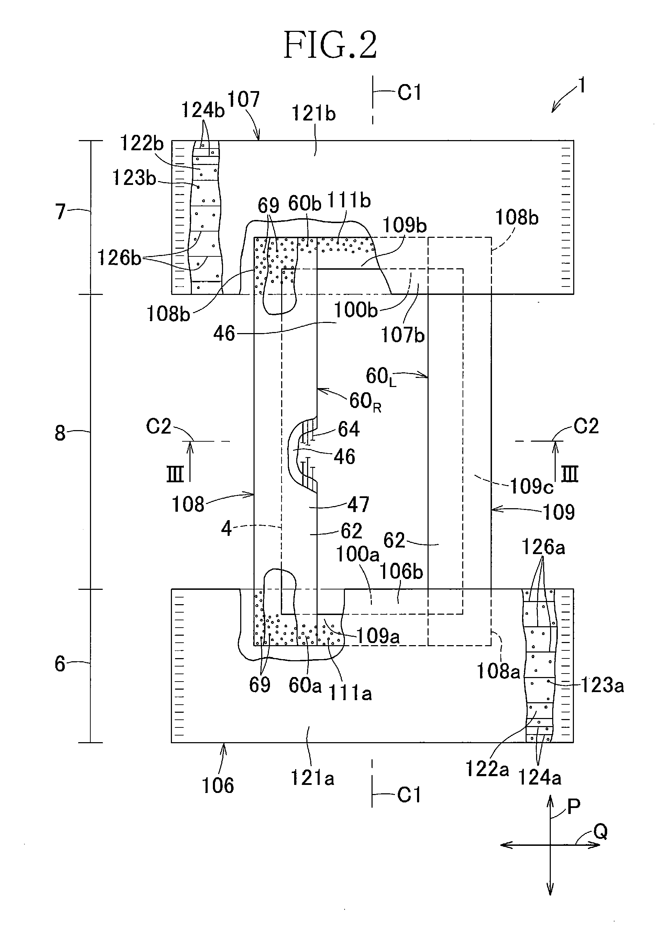

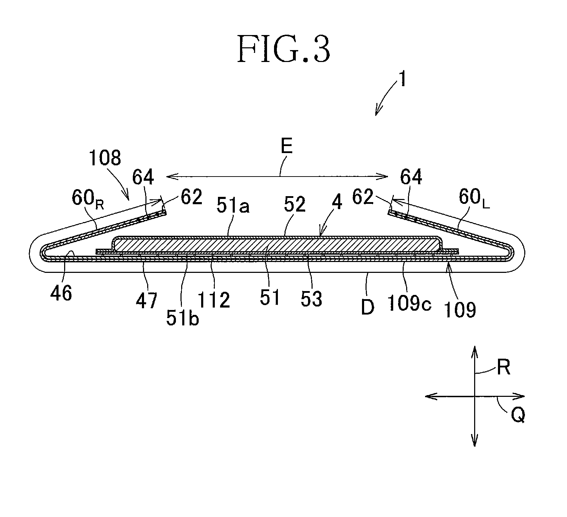

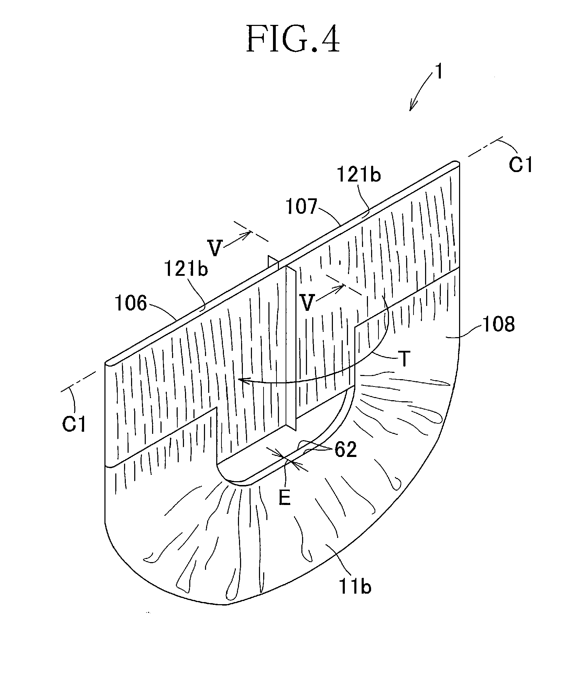

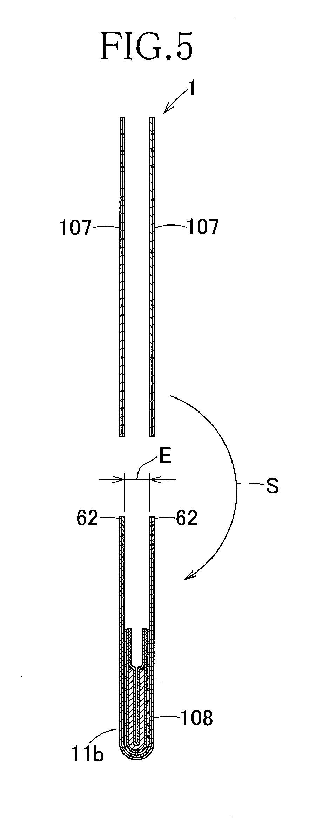

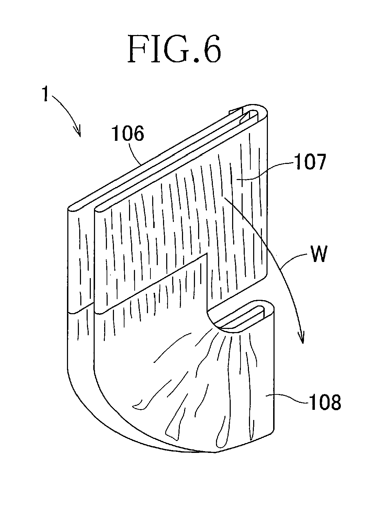

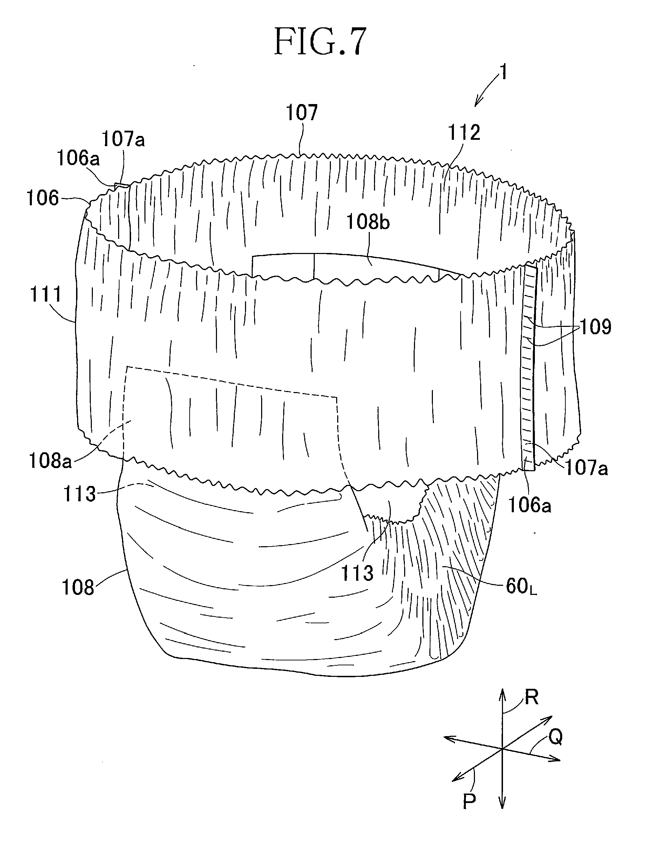

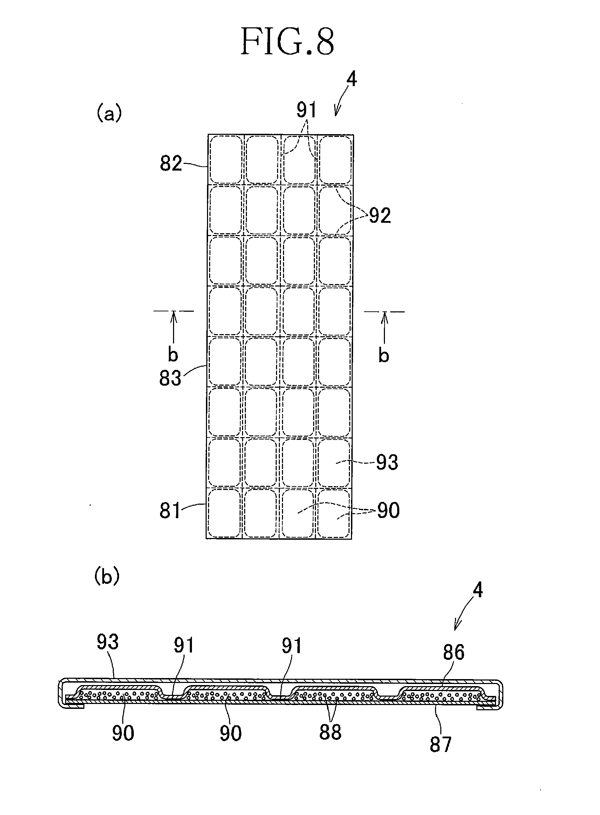

{Fig. 1} Fig. 1 is a partially cutaway perspective view of a disposable pants-type wearing article (disposable pants-type diaper).
{Fig. 2} Fig. 2 is a partially cutaway plan view illustrating the diaper in a flatly developed state.
{Fig. 3} Fig. 3 is a sectional view taken along line III-III of Fig. 2.
{Fig. 4} Fig. 4 is a perspective view illustrating the diaper in a folded state.
{Fig. 5} Fig. 5 is a sectional view taken along line V-V in Fig. 4.
{Fig. 6} Fig. 6 is a perspective view illustrating another example of a fold pattern of the diaper.
{Fig. 7} Fig. 7 is a partially cutaway perspective view similar to Fig. 1, illustrating another embodiment of the disposable pants-type wearing article (disposable pants-type diaper).
{Fig. 8} Fig. 8 (a) is a plan view of a bodily fluid absorbent structure and Fig. 8 (b) is a sectional view taken along line b-b in Fig. 8 (a) .

### {Description of Embodiments}

Details of the disposable pants-type wearing article according to this invention will be described hereunder with reference to the accompanying drawings.

Fig. 1 is a partially cutaway perspective view of a disposable pants-type diaper as one embodiment. The diaper 1 includes a front panel 106 defining a front waist region 6, a rear panel 107 defining a rear waist region 7 and a central panel 108 defining a crotch region 8. The front panel 106 and the rear panel 107 are put flat along respective opposite side edges 106a, 107a and joined together at a pair of series of seams 109 formed in a vertical direction to form an annular waist region 111 and to define a waist-opening 112. An inner surface of a front end 108a of the central panel 108 is joined to an outer surface of the front panel 106. An inner surface of a rear end 108b (See Fig. 2) is joined to an outer surface of the rear panel 107. These central panel 108 and the front and rear panels 106, 107 define apairof leg-openings 113. In Fig. 1, afront-backdirection, a transverse direction and the vertical direction are respectively indicated by double-headed arrows P, Q, R together with a center line C1 bisecting a dimension of the diaper 1 in the transverse direction Q.

Fig. 2 is a partially cutaway plan view illustrating the diaper 1 in a state after the front panel 106, the rear panel 107 and the central panel 108 have been flatly developed after the front panel 106 and the rear panel 107 were unjoined at the seams 109 on the both sides. The front-back direction and the transverse direction of the diaper 1 are the same as the front-back direction P and the transverse direction Q of the diaper 1 in Fig. 1.

The front panel 106 in the diaper 1 includes an inner sheet 121a and an outer sheet 122a permanently bonded to each other with a hot melt adhesive 123a. A plurality of waist- elastic members 124a and a plurality of auxiliary elastic members 126a extending in parallel to each other in the transverse direction Q are interposed between the inner sheet 121a and the outer sheet 122a and bonded under tension at least to one of the inner sheet 121a and the outer sheet 122a with a hot melt adhesive (not shown) . The rear panel 107 includes an inner sheet 121b and an outer sheet 122b permanentlybonded to each other with a hot melt adhesive 123b. A plurality of waist elastic members 124b and a plurality of auxiliary elastic members 126b extending in parallel to each other in the transverse direction Q are interposed between the inner sheet 121b and the outer sheet 122b and bonded under tension at least to one of the inner sheet 121b and the outer sheet 122b with a hot melt adhesive (not shown). In the front panel 106 and the rear panel 107, as material of the inner sheets 121a, 121b and the outer sheets 122a, 122b, for example, a nonwoven fabric formed of thermoplastic synthetic fibers, a liquid-impervious and a moisture-pervious plastic film or a laminate of these nonwoven fabric and film may be used.

The central panel 108 in the diaper 1 includes a mount portion 109 formed from an inner sheet 46 and an outer sheet 47 bonded to each other with a hot melt adhesive (not shown), a bodily fluid absorbent structure 4 attached to the inner sheet 4 and defining a bodily fluid absorbent region and a pair of barrier flaps 60L, 60R. The mount portion 109 has a front end segment 109a, a rear end segment 109b and an intermediate segment 109c extending between these opposite end segments 109a, 109b. The barrier flaps 60L, 60R are formed by folding respective extensions of the inner sheet 46 and the outer sheet 47 bonded to each other and extending outward in the transverse direction Q beyond the bodily fluid absorbent structure 4 in such a manner that the inner sheet 46 may lie on the inner side. Segments of the inner sheet 46 cooperating with segments of the outer sheet 47 to define the barrier flaps 60L, 60R are bonded to the segments of the inner sheet 46 cooperating with segments of the outer sheet 47 to define the front and rear end segments 109a, 109b with a hot melt adhesive 69. The barrier flaps 60L, 60R respectively have proximal side edges 62 adapted to come in contact with skin of a wearer (not shown) of the diaper 1 and, in each of the proximal side edges 62, apluralityof elasticmembers 64 interposedbetween the inner sheet 46 and the outer sheet 47 and secured to at least one of these sheets 46, 47 with a hot melt adhesive (not shown) so that each of the proximal side edges 62 may come close to or in contact with the wearer's skin under contraction of the elastic members 64. In Fig. 1, only the barrier flaps 60L of such barrier flaps 60L, 60R is illustrated.

In such central panel 108, the front end segment 109a of the mount portion 109 and respective front end segments 60a of the barrier flaps 60L, 60R are bonded to the outer surface of the outer sheet 122a of the front panel 106 with a hot melt adhesive 111a . In a similar fashion, the rear end segment 109b and respective rear end segments 60b of the barrier flaps 60L, 60R are bonded to the outer sheet 122b of the rear panel 107 with a hot melt adhesive 111b. In this regard, an inner end segment 106b of the front panel 106 is not bonded to the mount portion 109 of the central panel 108 and, in consequence, a pocket 100a is defined between the front panel 106 and the central panel 108 . In a similar fashion, an inner end segment 107b of the rear panel 107 is not bonded to the mount portion 109 and, in consequence, a pocket 100b is defined between the rear panel 107 and the central panel 108. When bodily fluids flow upward along the side of the inner surface of the central panel 108, these pockets 100a, 100b are capable of receiving such bodily fluids and preventing leak thereof.

Fig. 3 is a sectional view taken along line III-III in Fig. 2 and line III-III extends across an intermediate segment 109c of the mount portion 109 of the central panel 108 in the transverse direction Q so as to overlap with a transverse center line C2. In a cross-section of Fig. 3, the central panel 108 has a length D extending along the outer sheet 47 from the proximal side edge 62 of the barrier flap 60L to the proximal side edge 62 of the barrier flap 60R and a distance E between the opposite proximal side edges 62. On the assumption that the diaper 1 is for adult, preferred length D is in a range of 275 to 700 mm and a preferred distance E is in a range of 30 to 230 mm. The inner sheet 46 is preferably water-impervious and may be formed of a soft, liquid-impervious and moisture-pervious plastic film such as a low density polyethylene film having a thickness in a range of 5 to 20 umor a nonwoven fabric formed of thermoplastic synthetic fibers having a fineness in a range of 0.1 to 5 dtex and a mass per unit area in a range of 10 to 30 g/m². The outer sheet 47 is preferably formed of a nice and soft nonwoven fabric made of thermoplastic synthetic fibers having a fineness in a range of 0.1 to 5 dtex and a mass per unit area in a range of 8 to 20 g/m².

The bodily fluid absorbent structure 4 in the form of a pad includes an absorbent core 51, a water-pervious inner sheet 52 covering an inner surface 51a of the absorbent core 51 and a water-impervious outer sheet 53 covering an outer surface 51b of the absorbent core 51. The water-pervious inner sheet 52 and the water-impervious outer sheet 53 extend outward in both the front-back direction P and the transverse direction Q beyond the peripheral edge of the water-absorbent core 51 to have respective extensions which are, in turn, put flat together and bonded to each other with a hot melt adhesive (not shown) . This water-pervious inner sheet 52 constitutes part of the inner surface of the central panel 108 adapted to face the wearer's skin. The water-impervious outer sheet 53 is bonded to the inner sheet 46 with a hot melt adhesive 112. While the absorbent core 51 formed in this manner has a stiffness higher than that of the laminate sheet of the inner sheet 46 and the outer sheet 47, the absorbent core 51 is sufficiently flexible to conform with the wearer's movements and to fit the wearer's body. When a water-impervious sheet is used as material of the inner sheet 46, it is also possible to use a water-pervious sheet may be used as the outer sheet 53.

As material of the absorbent core 51 in such a bodily fluid absorbent structure 4, it is possible to use water-insoluble and water swellable superabsorbent polymer particles having a water-absorbing capacity corresponding to several dozen times of own mass at a rate of 50 to 150 g/m² or fluff pulp at a rate of 70 to 150 g/m² or a mixture of superabsorbent polymer particles and wood fluff pulp. Optionally, thermoplastic staple fibers of 15% by mass may be mixed into the above-mentioned material. As material of the water-pervious inner sheet 52 adapted to wrap the absorbent core 51, a nonwoven fabric formed of thermoplastic synthetic fibers having a fineness of 0.1 to 5 dtex and a mass per unit area in a range of 10 to 50 g/m² may be used in the form of a single sheet or a plurality of sheets layered one another. As material of the water-impervious outer sheet 53, a moisture-pervious plastic film having a thickness of 5 to 20 um may be used and a substantially water-impervious nonwoven fabric also may be used. When the nonwoven fabric formed of thermoplastic synthetic fibers is used as the water-pervious inner sheet 52, preferably this nonwoven fabric is previously treated to become hydrophilic.

Referring to Figs. 4 and 5, Fig. 4 is a perspective view of the diaper 1 of Fig. 1 having the front panel 106 and the rear panel 107 folded on themselves, respectively, so that the inner sheet 121a of the front panel 106 and the inner sheet 121b of the rear panel 102 folded along the center line C1 may face themselves, respectively, and Fig. 5 is a sectional view taken along line V-V in Fig. 4. In the flexible diaper 1 having the length D in the central panel 108 dimensioned to be sufficiently long to facilitate the bodily fluid absorbent structure 4 to be deformed in the transverse direction B, with this diaper 1 held by both hands (not shown) , the front panel 106 and the rear panel 107 may be folded to the state as illustrated to bring the central panel 108 into its state hanging down from the front panel 106 and the rear panel 107. In this state, the proximal side edges 62 of the barrier flaps 60L, 60R defined by both side edge portions of the central panel 108 get close to each other together with the vicinities of the respective proximal side edges 62 in the width direction Q and, in this way, the distance E between the opposite proximal side edges 62 (See Fig. 3) may be reduced to a range of 0 to 50 mm. In this regard, the distance E = 0 means that the respective proximal side edges 62, 62 are in contact with each other. When the used diaper 1 soiled with body waste is in this state, the front panel 106, the rear panel 107 and the central panel 108 hanging down from these front and rear panels 106, 107 cooperate together to define a bag and body waste is collected on a bottom 11b of this bag. The proximal side edges 62, 62 defined by the opposite side edges of the central panel 108 are in contact with or closest to each other and thereby behave to close an opening of the bag to prevent body waste from leaking out of the inner side of the central panel.

When the diaper 1 illustrated in Fig. 5 is folded in a direction indicated by an arrow S, i.e., the diaper 1 is folded so that the front panel 106 and the rear panel 107 may overlap the central panel 108, the proximate side edges 62, 62 are put in close contact with each other to improve the preventive effect against leakage of body waste and, at the same time and to downsize a bulk of the used diaper 1, and thereby facilitate disposal of the used diaper.

Fig. 6 is a perspective view illustrating another example of a fold pattern of the diaper 1 distinguished from the fold pattern illustrated in Fig. 5. It is also possible to overlap the opposite proximal side edges 62, 62 each other and, at the same time, to downsize a bulk of the used diaper 1 by folding the used diaper 1 in a direction indicated by an arrow T. It is also possible to fold the diaper 1 illustrated in Fig. 6 in a direction indicated by an arrow W.

Fig. 7 is a perspective view illustrating the disposable pants-type diaper 1 according to an embodiment distinguished from the embodiment illustrated in Fig. 1. In the diaper 1 illustrated in Fig. 7, the front end segment 108a of the central panel 108 is bonded to the inner surface of the front panel 106 and the rear end segment 108b is bonded to the inner surface of the rear panel 107. Also in such embodiment, the used diaper 1 may be folded in fold patterns similar to those illustrated in Figs. 4 through 6 for disposal thereof. Though not shown, this invention includes an embodiment according to which the central panel 108 has its front end segment 108a bonded to the inner surface of the front panel 106 and the rear end segment 108b bonded to the inner surface of the rear panel 107, and an embodiment according to which the central panel 108 has its front end segment 108a bonded to the outer surface of the front panel 106 and its rear end segment 108b bonded to the inner surface of the rear panel 107 and vice versa.

Fig. 8(a) and Fig. 8(b) illustrate an example of the bodily fluid absorbent structure 4 wherein Fig. 8(a) is a plan view of the bodily fluid absorbent structure 4 and Fig.8(b) is a sectional view taken along line b-b in Fig. 8(a).

This bodily fluid absorbent structure 4 is the structure which may be used also as the bodily fluid absorbent structure 4 illustrated in Fig. 3 and has a front end segment 81, a rear end segment 82 and an intermediate segment 83 as viewed in a length direction thereof. In the front end segment 81, a second sheet
87 described below is attached to the inner surface of the front end segment 109a of the mount portion 109 in the central panel 108 illustrated in Fig. 2 with a hot melt adhesive. In the rear end segment 82, the second sheet 87 described below is attached to the inner surface of the rear end segment 109b of the mount portion 109 with hot melt adhesive. The intermediate segment 83 lies in the intermediate segment 109c of the mount portion 109 and, in this intermediate segment 83, the second sheet described below 87 is not bonded to the intermediate segment 109c or bonded to part of the intermediate segment 109c with hot melt adhesive. Referring to Fig. 8 (b), the bodily fluid absorbent structure 4 is defined by a water-pervious first sheet 86, the water-pervious second sheet 87 and superabsorbent polymer particles 88 interposed between these first and second sheets 86, 87 wherein the first sheet 86 and the second sheet 87 are heat sealed or adhesive-bonded to each other along a plurality of lines 91, 92 extending in the front-back direction and in the width direction intersecting the front-back direction, respectively, as will be seen in Fig. 8 (a). The first sheet 86 is covered with a nice and soft water-pervious third sheet 93 which is, in turn, folded along the peripheral edge of the bodily fluid absorbent structure 4 so as to overlap the second sheet 87. The respective portions of the second sheet 87 and the third sheet 93 overlapping each other are bonded to each other with a hot melt adhesive (not shown).

The first sheet 86 and the second sheet 87 joined to each other along the lines 91 and the lines 92 define a plurality of cells 90 each enveloping therein a required amount of superabsorbent polymer particles 88. An upper limit of the required amount of superabsorbent polymer particles 88 is set so that superabsorbent polymer particles 88 may freely move within the individual cells 90 so long as superabsorbent polymer particles 88 still not absorb water at all and the bodily fluid absorbent structure 4 may be easily folded on itself in the width direction Q, for example, the bodily fluid absorbent structure 4 may be very easily folded on itself along the center line C1 of the diaper 1.

In the bodily fluid absorbent structure 4 constructed in this manner, bodily fluids may pass not only through the first sheet 86 but also through the second sheet 87 into the cells 90. When it is tried to fold back the used diaper 1 on itself as illustrated in Fig. 4, superabsorbent polymer particles 88 are freelymovable, assuring the used diaper 1 to be smoothly folded on itself and, in consequence, the opposite side edge portions of the central panel 108 may move to come closer to each other without any interference due to the presence of superabsorbent polymer particles.

In the bodily fluid absorbent structure 4 illustrated in Fig. 8 as an example, as material of the first sheet 86 and the second sheet 87, a melt blown nonwoven fabric previously treated to become hydrophilic and having a mass per unit area in a range of 8 to 20 g/m² may be used and, as material of the third sheet 93, a spun bonded nonwoven fabric previously treated to become hydrophilic and having a mass per unit area in a range of 15 to 25 g/m² may be used. The third sheet 93 is used so as to face the wearer's skin and defines part of the inner surface of the central panel 108 in the diaper 1.

### {Reference Signs List}

- 1: disposable pants-type wearing article (disposable pants-type diaper)
- 4: bodily fluid absorbent structure
- 6: front waist region
- 7: rear waist region
- 8: crotch region
- 51: absorbent core
- 52: inner sheet
- 62: side edges (proximal side edges)
- 106: front panel
- 107: rear panel
- 108: central panel

## Claims

1. A disposable pants-type wearing article having a front-back direction, a transverse direction and a vertical direction being orthogonal to each other, the article including:
a front panel defining a front waist region, a rear panel defining a rear waist region and a central panel defining a crotch region, wherein these panels are cooperating together to define a pants shape, wherein
each of the panels having an inner surface and an outer surface opposite to the inner surface,
end segments of the central panel opposite in the front-back direction being bonded to the front panel and the rear panel, respectively, and
the central panel includes a bodily fluid absorbent structure, wherein:
in the opposite end segments of the central panel, opposite side edge portions of the central panel are folded with the inner surface lying inside and bonded to itself;
the respective end segments are at least partially bonded to the front panel and the rear panel, respectively; and
when the front panel and the rear panel are respectively folded along a center line bisecting a dimension in the transverse direction of the wearing article with the inner surfaces lying inside, the opposite side edges of the central panel get close to each other in a midsection of the crotch region until a distance between respective side edges of the opposite side edges is reduced to a range of 0 to 50 mm.

2. The wearing article according to claim 1, wherein when the front panel and the rear panel are respectively folded along the center line with the inner surfaces lying inside, the respective inner surfaces of the opposite side edge portions of the central panel overlap each other in the crotch region.

3. The wearing article according to claim 1 or 2, wherein the central panel is bonded to the outer surface of at least one of the front and rear panels.

4. The wearing article according to claim 3, wherein the central panel bonded to the outer surface has an intermediate segment as viewed in the transverse direction not bonded to a lower segment as viewed in the vertical direction of at least one of the front and rear panels and this intermediate segment cooperates with at least one of the front and rear panels to define a pocket opening downward as viewed in the vertical direction.

5. The wearing article according to claim 1 or 2, wherein the central panel is bonded to the respective inner surfaces of the front and rear panels.

6. The wearing article according to any one of claims 1 through 5, wherein a distance between the opposite side edges extending along the outer surface of the central panel in the midsection of the crotch region is in a range of 275 to 700 mm.

7. The wearing article according to any one of claims 1 through 6, wherein the bodily fluid absorbent structure includes an absorbent core in the form of a pad and a water-pervious inner sheet covering at least the upper surface thereof and the absorbent core includes at least superabsorbent polymer particles.
